(19) Europäisches Patentamt
European Patent Office
Office européen des brevets

(11) EP 2 113 243 A1

(12) # DEMANDE DE BREVET EUROPEEN

(43) Date de publication:
**04.11.2009 Bulletin 2009/45**

(51) Int Cl.:
***A61K 8/73*** *(2006.01)* ***A61K 8/87*** *(2006.01)*
***A61Q 5/04*** *(2006.01)*

(21) Numéro de dépôt: **09158013.4**

(22) Date de dépôt: **16.04.2009**

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **28.04.2008 FR 0852854**

(71) Demandeur: **L'Oreal**
**75008 Paris (FR)**

(72) Inventeurs:
- **Pasquet, Dorothée**
**92270, Bois Colombes (FR)**
- **Laurent, Ludivine**
**28000 Chartres (FR)**

(74) Mandataire: **Dossmann, Gérard**
**Bureau D.A. Casalonga & Josse**
**Bayerstrasse 71/73**
**80335 München (DE)**

(54) **Composition cosmétique comprenant un polyuréthane cationique et un dérivé de cellulose et utilisations en coiffage**

(57) La présente demande concerne une cosmétique comprenant, dans un milieu cosmétiquement acceptable :
(i) un ou plusieurs polyuréthanes cationiques comportant au moins un motif non ionique dérivé d'un homo- et/ou copolymère d'oléfine, et
(ii) une ou plusieurs celluloses particulières.

La présente demande concerne aussi les utilisations de cette composition.



Printed by Jouve, 75001 PARIS (FR)

## Description

La présente invention concerne de nouvelles compositions cosmétiques comprenant au moins un polyuréthane cationique comportant des motifs non ioniques dérivés d'au moins un homopolymère ou copolymère d'oléfine, et au moins une cellulose cationique ou non ionique.

La formation de dépôts et de films à propriétés élastiques fait depuis toujours l'objet d'importantes recherches en cosmétique. En effet, la plupart des zones du corps humain susceptibles de recevoir des dépôts cosmétiques, telles que la peau, les lèvres, les cheveux, les cils et les ongles sont soumises à des déformations et contraintes mécaniques importantes. Les films et dépôts cosmétiques doivent pouvoir résister à ces contraintes et suivre ces déformations sans se casser.

L'utilisation de polyuréthanes en cosmétique est connue depuis longtemps et est décrite par exemple dans les brevets WO94/13724 et EP0619111.

Les polyuréthanes décrits dans ces documents forment des films cassants inacceptables pour une application cosmétique.

Il existe certes d'autres polymères physiologiquement acceptables comme par exemple les polymères acryliques, mais ces polymères forment généralement des dépôts très collants, ce qui représente un inconvénient dans la plupart des applications cosmétiques.

L'utilisation des polyuréthanes cationiques à caractère élastique dans des compositions cosmétiques, notamment des compositions de coiffage, est connue.

Ainsi la demande de brevet français FR 2 815 350 décrit des polyuréthanes cationiques à caractère élastique et leur utilisation pour la formulation de laques et de compositions de coiffage améliorant la souplesse de la coiffure, c'est-à-dire permettant d'obtenir une tenue des cheveux plus naturelle que celle obtenue avec des polymères fixants usuels.

La demande de brevet français FR-2 833 960 décrit des compositions cosmétiques de coiffage, et en particulier des compositions de coiffage à rincer, notamment des shampoings coiffants, comportant un polyuréthane cationique ou amphotère auto-adhésif.

Cependant il a été observé que les produits de coiffage comprenant de tels polyuréthanes cationiques élastiques sont peu compatibles avec des agents épaississants ou gélifiants classiques, les gels obtenus avec ces polyuréthanes présentent une viscosité généralement faible qui ne permet pas d'obtenir la mise en forme souhaitée.

De manière surprenante, la Demanderesse a découvert que des compositions cosmétiques comprenant un ou plusieurs polyuréthanes cationiques comportant des motifs dérivés d'un homo- et/ou copolymère d'oléfine et une ou plusieurs celluloses cationiques ou non ioniques conduisent à l'obtention de gels de viscosité satisfaisante permettant une application facile sur les matières kératiniques

Ces gels permettent une mise en forme des cheveux rapide et facile, ils permettent ainsi d'obtenir des coiffures plus résistantes aux sollicitations mécaniques, à l'eau et à l'humidité, par rapport aux compositions à base de polyuréthanes mentionnées ci-dessus.

En outre, le maintien obtenu est également encore plus naturel.

Les gels obtenus présentent de bonnes propriétés fixantes et aussi permettent l'obtention de coiffures flexibles, la mise en forme de la coiffure est maintenue sur une longue durée.

La présente invention a par conséquent pour objet une composition cosmétique comprenant, dans un milieu aqueux cosmétiquement acceptable :

(i) un ou plusieurs polyuréthanes cationiques comportant un ou plusieurs motifs non ioniques dérivés d'un homo-et/ou copolymère d'oléfine, et
(ii) une ou plusieurs celluloses cationiques ou non ioniques.

D'autres caractéristiques, propriétés et avantages de la présente invention apparaîtront plus clairement à la lecture de la description et des exemples qui suivent.

De préférence la composition selon l'invention est telle que 50% en poids ou plus des motifs non ioniques du polyuréthane dérivent d'un homopolymère ou copolymère d'oléfine.

Les polyuréthanes cationiques préférés convenant pour l'invention contiennent :

(a) des motifs cationiques dérivés d'un ou plusieurs composés, de préférence une amine tertiaire ou quaternaire, présentant au moins deux fonctions réactives à hydrogène labile,
(b) des motifs non ioniques dérivés de polymères non ioniques portant à leurs extrémités des fonctions réactives à hydrogène labile, au moins un des motifs (b), de préférence au moins 50% en poids des motifs (b) et mieux la totalité des motifs (b) étant un motif ou des motifs (b1) dérivant d'un homo- ou copolymère d'oléfine portant à ses extrémités des fonctions réactives à hydrogène labile, et

(c) des motifs dérivés d'un ou plusieurs diisocyanates.

Plus particulièrement les polyuréthanes cationiques préférés comprennent :

(a) des motifs cationiques résultant de la réaction d'une ou plusieurs amines tertiaires ou quaternaires présentant au moins deux fonctions réactives à hydrogène labile,
(b) des motifs non ioniques dont au moins un motif (b1) résulte de la réaction d'au moins un polymère choisi parmi les homopolymères et les copolymères d'oléfines portant à leurs extrémités des fonctions réactives à hydrogène labile et ayant une température de transition vitreuse (Tg), mesurée par analyse enthalpique différentielle, inférieure à 10°C, et
(c) des motifs résultant de la réaction d'un ou plusieurs diisocyanates.

Par motif cationique, on entend, au sens de la présente invention, tout motif qui, soit par sa nature chimique propre, soit en fonction du milieu et/ou du pH dans lequel il se trouve, sera sous forme cationique.
On entend par fonctions réactives à hydrogène labile des fonctions capables, après départ d'un atome d'hydrogène, de former des liaisons covalentes avec les fonctions isocyanate des composés formant les motifs (c). On peut citer à titre d'exemple de telles fonctions les groupes hydroxyle, amine primaire ($-NH_2$) ou amine secondaire (-NHR), ou encore les groupes thiol (-SH).
La polycondensation de composés portant ces fonctions réactives à hydrogène labile avec des diisocayanates donne, selon la nature des fonctions réactives portant l'hydrogène labile (-OH, $-NH_2$, - NHR ou -SH), respectivement des polyuréthanes, des polyurées ou des polythio-uréthanes. Tous ces polymères sont regroupés dans la présente demande, par souci de simplification, sous le terme de polyuréthanes. De préférence, les polymères de l'invention sont de vrais polyuréthanes : les fonctions réactives portant l'hydrogène labile sont des fonctions OH.
Lorsque les amines tertiaires ou quaternaires formant les motifs (a) portent plus de deux fonctions à hydrogène labile les polyuréthanes obtenus présentent une structure ramifiée.
Dans un mode de réalisation préféré des polyuréthanes de la présente invention, les amines tertiaires ou quaternaires formant les motifs cationiques (a) ne présentent que deux fonctions réactives à hydrogène labile et les polyuréthanes obtenus par polycondensation ont par conséquent une structure essentiellement linéaire.
Il est bien entendu également possible d'utiliser un mélange d'amines difonctionnelles contenant une faible proportion d'amines portant plus de deux fonctions réactives à hydrogène labile.
Les amines tertiaires ou quaternaires formant les motifs cationiques (a) sont de préférence choisies parmi les composés correspondant à l'une des formules suivantes :

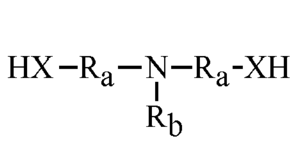

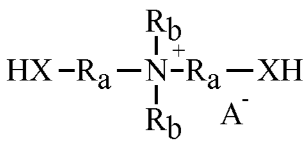

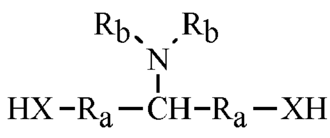

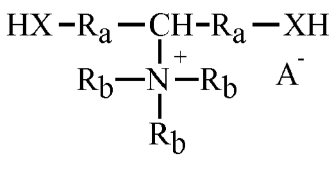

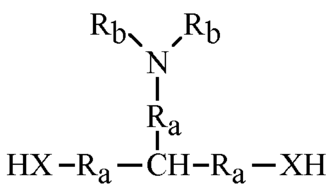

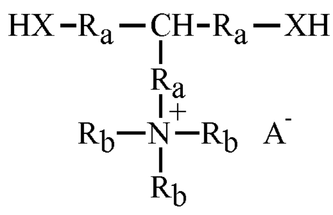

dans lesquelles
chaque $R_a$ représente indépendamment un groupe alkylène en $C_{1-6}$, linéaire ou ramifié, cycloalkylène en $C_{3-6}$ ou arylène, tous pouvant être substitués par un ou plusieurs atomes d'halogène et comporter un ou plusieurs hétéroatomes choisis parmi O, N, P et S,
chaque $R_b$ représente indépendamment un groupe alkyle en $C_{1-6}$, cycloalkyle en $C_{3-6}$ ou aryle, tous pouvant être substitués par un ou plusieurs atomes d'halogène et comporter un ou plusieurs hétéroatomes choisis parmi O, N, P et S,
chaque X représente indépendamment un atome d'oxygène ou de soufre ou un groupe NH ou $NR_c$, où $R_c$ représente un groupe alkyle en $C_{1-6}$, et
$A^-$ représente un contre-ion physiologiquement acceptable.

On peut citer à titre d'amines tertiaires particulièrement préférées pour l'obtention des polyuréthanes cationiques, la N-méthyldiéthanolamine et la N-tert-butyldiéthanolamine.

Les amines tertiaires et quaternaires formant les motifs cationiques (a) des polyuréthanes de la présente invention peuvent également être des polymères à fonction(s) amine tertiaire et/ou quaternaire, portant à leurs extrémités des fonctions réactives à hydrogène labile. La masse molaire moyenne en poids de ces polymères à fonctions amine tertiaire et/ou quaternaire est de préférence comprise entre 400 et 10 000.

On peut citer à titre d'exemples de tels polymères à fonctions amine appropriés les polyesters issus de la polycondensation de la N-méthyldiéthanolamine et de l'acide adipique.

Lorsque les amines formant les motifs cationiques (a) sont des composés à fonction(s) amine tertiaire, une partie ou la totalité de ces fonctions amine doit être neutralisée par un agent de neutralisation approprié choisi parmi les acides organiques ou minéraux physiologiquement acceptables. On peut citer à titre d'exemple d'acides préférés l'acide chlorhydrique ou l'acide acétique.

Le deuxième type de motifs formant les polyuréthanes préférés de la présente invention sont des motifs macromoléculaires, appelés motifs (b), dérivés de polymères non ioniques portant à leurs extrémités des fonctions réactives à hydrogène labile et ayant de préférence une température de transition vitreuse (Tg), mesurée par analyse enthalpique différentielle, inférieure à 10°C.

Selon l'invention, au moins un de ces motifs (b1) dérive d'un homo- ou copolymère d'oléfine.

Les propriétés viscoélastiques des polyuréthanes sont particulièrement avantageuses lorsque les motifs (b) sont dérivés de polymères ayant une température de transition vitreuse inférieure à 0°C et mieux encore inférieure à -10°C.

Ces polymères ont de préférence une masse molaire moyenne en poids comprise entre 400 et 10 000 et plus particulièrement entre 1000 et 5000.

Les polymères non ioniques susceptibles de former les motifs non ioniques (b2) différents des motifs non-ioniques (b1) dérivant des homo- et copolymères d'oléfine, peuvent être choisis parmi les polyéthers, les polyesters, les polysiloxanes, les polycarbonates et les polymères fluorés.

De préférence, les polymères susceptibles de former les motifs non-ioniques (b) sont choisis uniquement parmi les homo- et copolymères d'oléfine.

Parmi les polymères d'oléfine terminés par des groupes réactifs à hydrogène labile convenant pour la présente invention on peut citer les homopolymères et les copolymères statistiques ou blocs d'éthylène, de propylène, 1-butylène, 2-butylène, isobutylène, 1,2-butadiène, 1,4-butadiène et d'isoprène et leurs mélanges.

Les homo- et copolymères de butadiène et d'isoprène peuvent être partiellement ou totalement hydrogénés.

Les polymères préférés sont les copolymères d'éthylène/butylène, les polybutadiènes et les polybutadiènes hydrogénés porteurs de groupes réactifs à hydrogène labile à leurs extrémités et en particulier de groupes hydroxyles. Encore plus préférentiellement ces polymères sont des 1,2 et/ou 1,4 polybutadiènes.

De tels polymères sont disponibles commercialement par exemple sous la dénomination KRATON® L, en particulier KRATON® L 2203 (polybutadiènediol hydrogéné) auprès de la société KRATON polymers, KRASOL LBH® et LBHP®, notamment KRASOL LBHP® 2000 (polybutadiène diol) auprès de la société SARTOMER et GI® 3000 (copolymère éthylène/butylène) auprès de la société NISSO CHEMICAL.

Les diisocyanates formant les motifs (c) englobent les diisocyanates aliphatiques, alicycliques ou aromatiques.

Des diisocyanates préférés sont choisis parmi le méthylènediphényldiisocyanate, le méthylènecyclohexanediisocyanate, l'isophoronediisocyanate, le toluènediisocyanate, le natphtalènediisocyanate, le 1,4-butanediisocyanate et le 1,6-hexanediisocyanate. Ces diisocyanates peuvent bien entendu être utilisés seuls ou sous forme de mélange de deux ou plusieurs diisocyanates. Encore plus préférentiellement le diisocyanate est l'isophoronediisocyanate.

Comme indiqué ci-avant, les polyuréthanes cationiques de la présente invention peuvent contenir, en plus des motifs (a), (b1) et (c), une certaine fraction de motifs (b2) dérivés de composés, en général monomères, non ioniques contenant au moins deux fonctions à hydrogène labile et différents des composés conduisant aux motifs (b1).

Ces motifs (b2) sont dérivés en général de diols en $C_1$-$C_{12}$, par exemple de néopentylglycol, d'hexaéthylèneglycol, de 1,2-éthanediol, de 1,2-propanediol et de 1,3-propanediol ou d'aminoalcools, en $C_1$-$C_6$ par exemple l'aminoéthanol.

Les polyuréthanes cationiques de l'invention présentent de préférence un caractère élastique.

Dans un mode de réalisation particulier de l'invention, le polyuréthane cationique ne comprend pas de motifs en plus des motifs (a), (b) et (c). Un polyuréthane répondant à cette définition est le polyuréthane (1) décrit dans les exemples obtenu par polycondensation de KRASOL LBHP® 2000, de N-méthyldiéthanolamine et de isophoronediisocyanate.

Dans un mode de réalisation alternatif, le polyuréthane cationique comprend des motifs en plus des motifs (a), (b) et (c).

Un paramètre physique caractérisant les propriétés viscoélastiques des polyuréthanes cationiques ci-dessus est leur recouvrance en traction. Cette recouvrance est déterminée par essai de fluage en traction consistant à étirer rapidement une éprouvette jusqu'à un taux d'allongement prédéterminé, puis à relâcher la contrainte et à mesurer la longueur de l'éprouvette.

L'essai de fluage utilisé pour la caractérisation des polyuréthanes cationiques utilisés selon la présente invention se déroule de la manière suivante :

On utilise, comme éprouvette, un film du polyuréthane ayant une épaisseur de 500 ± 50 mm, découpé en bandes de 80 mm x 15 mm. Ce film de copolymère est obtenu par séchage, à une température de 22 ± 2°C et à une humidité relative de 50 ± 5 %, d'une solution ou dispersion à 3 % en poids dudit polyuréthane dans de l'eau et/ou de l'éthanol.

Chaque bande est fixée entre deux mors, distants de 50 ± 1 mm l'un de l'autre, et est étirée à une vitesse de 20 mm/minute (dans les conditions de température et d'humidité relative ci-dessus) jusqu'à un allongement de 50 % ($\varepsilon_{max}$), c'est-à-dire l'obtention d'une bande présentant jusqu'à 1,5 fois sa longueur initiale. On relâche alors la contrainte en imposant une vitesse de retour égale à la vitesse de traction, soit 20 mm/minute, et on mesure l'allongement de l'éprouvette (exprimé en % par rapport à la longueur initiale) immédiatement après retour à charge nulle ($\varepsilon_i$).

La recouvrance instantanée ($R_i$) est calculée à l'aide de la formule suivante :

$$R_i\ (\%) = ((\varepsilon_{max} - \varepsilon_i) / \varepsilon_{max})\ x\ 100$$

Les polyuréthanes cationiques de la présente invention ont de préférence une recouvrance instantanée ($R_i$), mesurée dans les conditions indiquées ci-dessus, comprise entre 5 % et 95 %, en particulier comprise entre 20 % et 90 % et idéalement entre 35 et 85 %.

La température de transition vitreuse (Tg) des polymères non ioniques formant les motifs (b) et des polyuréthanes cationiques de la présente invention est mesurée par analyse enthalpique différentielle (DSC, *differential scanning calorimetry*) selon la norme ASTM D3418-97.

Les polyuréthanes cationiques de la présente invention présentent de préférence au moins deux températures de transition vitreuse, dont une au moins est inférieure à 10°C, de préférence inférieure à 0°C et mieux encore inférieure à -10°C, et au moins une autre est supérieure ou égale à la température ambiante (20°C).

La recouvrance instantanée et par conséquent les propriétés viscoélastiques des polyuréthanes de la présente invention dépend des fractions des différents motifs monomères (a), (b1), (b2) et (c).

La fraction de motifs (a) doit être de préférence suffisante pour conférer aux polymères leur charge positive responsable de leur bonne affinité pour les substrats kératiniques. Les motifs (b) doivent de préférence représenter une fraction en poids suffisante pour que les polyuréthanes présentent de préférence au moins une température de transition vitreuse inférieure à 10 °C et ne forment pas des films cassants.

De manière préférée, les motifs (a) représentent de 0,1 à 90 %, de préférence de 1 à 30 %, mieux de 1 à 20% et idéalement de 5 à 10% en poids, les motifs (b1) de 10 à 99,9 %, de préférence de 20 à 99 % et mieux de 30 à 85% en poids et les motifs (b2) de 0 à 50 % en poids, de préférence de 0 à 30 % en poids par rapport au poids total des motifs du polyuréthane. De préférence encore les polyuréthanes de l'invention ne comportent pas de motif (b2).

De manière préférée les motifs (c) représentent de 1 à 60% mieux de 5 à 50% idéalement de 10 à 40% du poids total des motifs polyuréthane.

Les motifs (c) sont de préférence présents en une quantité essentiellement stoechiométrique par rapport à la somme des motifs (a) et (b). En effet, l'obtention de polyuréthanes ayant des masses molaires importantes suppose un nombre de fonctions isocyanate pratiquement identique au nombre de fonction à hydrogène labile. L'homme du métier saura choisir un éventuel excès molaire de l'un ou de l'autre type de fonction pour ajuster la masse molaire à la valeur désirée.

La quantité de polyuréthane présente dans une composition cosmétique objet de l'invention, dépend bien entendu du type de composition et des propriétés recherchées et peut varier à l'intérieur d'une gamme très large, comprise généralement de préférence entre 0,01 et 40 % en poids, de préférence entre 0,05 et 20 %, idéalement entre

0,1 et 10% en poids, rapporté à la composition cosmétique finale.

La cellulose est choisie parmi les celluloses cationiques et les celluloses non-ioniques.

Ces celluloses sont choisies parmi les celluloses ne comportant pas de chaîne hydrophobe et les celluloses comportant une ou plusieurs chaînes hydrophobes.

De préférence les celluloses de l'invention sont des éthers de celluloses. Encore plus préférentiellement ces celluloses sont des hydroxyalkylcelluloses et en particulier des hydroxyéthylcelluloses ou des hydroxypropylcelluloses. Elles peuvent ou non contenir une chaîne grasse. Parmi les celluloses non ioniques ne comportant pas de chaîne grasse, on peut citer les hydroxyéthylcelluloses, les hydroxypropylcelluloses, les hydroxypropylméthylcelluloses. Une hydroxypropylméthylcellulose particulièrement adaptée est le METHOCEL F4M proposé par DOW CHEMICALS (nom INCI HYDROXYPROPYLMETHYLCELLULOSE).

Plus particulièrement les celluloses cationiques peuvent être des dérivés d'éthers de cellulose comportant des groupements ammonium quaternaire tels que ceux décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium et correspondent au NOM INCI « POLYQUATERNIUM 10 ».

Les dérivés de cellulose cationiques peuvent aussi être des copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylamidopropyl triméthylammonium, de diméthyl-diallylammonium.

Les produits commercialisés répondant à cette définition sont plus particulièrement les produits vendus sous la dénomination "Celquat L 200" et "Celquat H 100" par la Société National Starch, ces produits correspondent à l'appellation INCI « POLYQUATERNIUM-4 ».

Les celluloses modifiées par des groupements comportant une ou plusieurs chaînes grasses peuvent être non-ioniques. Ce sont notamment :

- les hydroxyéthylcelluloses non-ioniques modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, comme le produit NATROSOL PLUS GRADE 330 CS® (alkyles en $C_{16}$) vendu par la société AQUALON correspondant à l'appellation INCI « CETYLHYDROXYETHYLCELLULOSE », ou le produit BERMOCOLL EHM 100® vendu par la société BEROL NOBEL,
- celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL correspondant à l'appellation INCI « NONOXYNYL HYDROXYETHYLCELLULOSE ».

Les celluloses modifiées par des groupements comportant une ou plusieurs chaînes grasses peuvent être cationiques. On peut notamment citer :

- ■ les hydroxyéthylcelluloses quaternisées par l'époxyde de lauryldiméthylammonium correspondant à l'appellation INCI « POLYQUATERNIUM-24 » telles que le produit QUADRISOFT POLYMER LM200 proposé par Amerchol,
- ■ les hydroxyéthylcelluloses modifiées par des chaînes grasses cationiques et par des groupes cationiques sans chaîne grasse correspondant à l'appellation INCI « POLYQUATERNIUM-67 » tels que les polymères proposés sous les appellations SOFTCAT SL-5, SL-30, SL-60, SL-100 par Amerchol.

La quantité de cellulose(s) dans une composition cosmétique selon l'invention va de 0,01 à 50 % en poids, de préférence de 0,1 à 20 %, encore plus préférentiellement de 0,5 à 10% en poids, rapporté au poids de la composition cosmétique finale.

De préférence, la composition selon l'invention comprend au moins un ester de polyéthylène glycol. De préférence cet ester de polyéthylène glycol est défini par la formule suivante :

$$X\text{-}(OCH_2CH_2)_mOY \qquad (I)$$

- m allant de 80 à 500 et
- X et Y représentant, indépendamment l'un de l'autre un atome d'hydrogène, ou un groupement acyle en $C_8$ à $C_{30}$ linéaire ou ramifié, saturé ou insaturé,

à condition que l'un au moins des groupements X et Y représente un groupement acyle.

De préference, du polyéthylène glycol comprenant 80 à 500 groupes d'oxyde d'éthylène par molécule est utilisé pour obtenir l'ester de polyéthylène glycol de formule (I) ci-dessus.

La masse moléculaire moyenne théorique Mth d'un polyéthylène glycol étant calculée d'après l'équation (I) :

$$Mth = 18 + 44xm \quad (I)$$

où m est tel que défini précédemment.

De préference m va de 80 à 350 et encore plus préférentiellement de 100 à 300.

De préférence, le rapport (R) :

$$R = \text{Masse de la partie hydrophile } (OCH_2CH_2)_m \text{ / (Masse de l'ester de polyéthylène glycol – masse de la partie hydrophile } (OCH_2CH_2)_m)$$

est compris entre 8 et 1000.

A titre d'ester de polyéthylène glycol de formule (I) on peut citer les monoesters de polyéthylène glycol tels que les produits référencés dans le « International Cosmetic Ingredient Dictionary and Handbook » 9ème édition, 2002, sous les références PEG90 STEARATE, PEG100 STEARATE, PEG120 STEARATE, PEG150 STEARATE, PEG150 LAURATE, et PEG150 OLEATE.

La quantité d'ester de polyéthylène glycol dans une composition cosmétique objet de l'invention, dépend bien entendu du type de composition et des propriétés recherchées et peut varier à l'intérieur d'une gamme très large, comprise généralement entre 0,01 et 50 % en poids, de préférence entre 0,1 et 20 %, encore plus préférentiellement entre 0,5 et 15% en poids, rapporté au poids de la composition cosmétique finale, étant entendu qu'un ou plusieurs types d'esters de polyéthylène glycol peuvent être incorporés à la composition cosmétique.

De préférence, le milieu cosmétiquement acceptable est aqueux.

La composition cosmétique selon l'invention peut comprendre en outre un ou plusieurs solvants organiques, de préférence dans une quantité comprise entre 0,05 et 40%, de manière tout à fait préférée, entre 1 et 20% en poids, par rapport au poids total de la composition.

Ce solvant organique peut être un alcool inférieur en $C_2$ à $C_4$, en particulier l'éthanol, un polyol tel que le propylène glycol ou le glycérol ou un éther de polyol.

Les compositions selon l'invention peuvent également contenir d'autres adjuvants cosmétiquement acceptables, tels que par exemple des agents tensioactifs, des agents épaississants différents des celluloses utilisées dans les compositions selon la présente demande, des agents co-épaississants, des agents de pénétration, des parfums, des colorants, des plastifiants, des tampons, et divers adjuvants usuels comme des cires, des silicones volatiles ou non, cycliques ou linéaires ou ramifiées, organomodifiées (notamment par des groupements amines) ou non, des céramides, des pseudocéramides, des huiles végétales, minérales ou de synthèse, les vitamines ou provitamines comme le panthénol, des opacifiants, des agents réducteurs, des émulsionnants, des conservateurs, des charges minérales, des nacres, des paillettes, des filtres solaires, des protéines, des polymères fixants anioniques, non ioniques, cationiques ou amphotères, des agents hydratants, des émollients, des agents adoucissants, des agents anti-mousse, des agents antiperspirants, des agents anti-radicaux libres, des bactéricides, des séquestrants, des anti-pelliculaires, des antioxydants, des agents alcalinisants, des acides, des parfums et tout autre additif classiquement utilisé dans les compositions cosmétiques destinées à être appliquées sur les cheveux.

Les agents tensioactifs utilisables dans la composition selon la présente invention peuvent être des tensioactifs anioniques, non ioniques, amphotères ou cationiques, ou des mélanges de ceux-ci.

Parmi les tensioactifs anioniques utilisables, seuls ou en mélanges, dans le cadre de la présente invention, on peut notamment citer les sels, et en particulier les sels de métaux alcalins tels que les sels de sodium, les sels d'ammonium, les sels d'amines, les sels d'amino-alcools ou les sels de métaux alcalino-terreux, par exemple, de magnésium, des composés suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkyl-arylsulfonates, les α-oléfine-sulfonates, les paraffine-sulfonates ; les alkylsulfosuccinates, les alkyléthersulfosuccinates, les alkylamidesulfosuccinates ; les alkylsulfoacétates ; les acylsarconisates ; et les acylglutamates, les groupes alkyle et acyle de tous ces composés comportant de 6 à 24 atomes de carbone et le groupe aryle désignant de préférence un groupe phényle ou benzyle.

On peut également utiliser dans le cadre de la présente invention les esters d'alkyle en $C_6$-$C_{24}$ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl ($C_6$-$C_{24}$)éther-carboxyliques polyoxyalkylénés, les acides alkyl($C_6$-$C_{24}$)aryl($C_6$-$C_{24}$)éther-carboxyliques polyoxyalkylénés, les acides alkyl($C_6$-$C_{24}$)amidoéther carboxyliques polyoxyalkylénés et leurs sels, en particulier ceux comportant de 2 à 50 groupements oxyde d'éthylène, et leurs mélanges.

Les agents tensioactifs non-ioniques que l'on peut utiliser dans le cadre de la présente invention, sont eux aussi, des composés bien connus en soi (voir notamment à cet égard "Handbook of Surfactants" par M.R. PORTER, éditions Blackie & Son (Glasgow and London), 1991, pp 116-178) Ils peuvent être notamment choisis parmi les alcools, les alpha-diols, les alkyl($C_1$-$C_{20}$) phénols ou les acides gras polyéthoxylés, polypropoxylés ou polyglycérolés, ayant une chaîne grasse comportant par exemple de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et d'oxyde de propylène, les condensats d'oxyde d'éthylène et d'oxyde de propylène sur des alcools gras ; les amides gras polyéthoxylés ayant de préférence de 2 à 30 moles d'oxyde d'éthylène ; les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acide gras du polyéthylèneglycol, les alkyl($C_6$-$C_{24}$)polyglucosides, les dérivés de N-alkyl($C_6$-$C_{24}$)glucamine, les oxydes d'amines tels que les oxydes d'alkyl($C_{10}$-$C_{14}$)amines ou les oxydes de N-acyl($C_{10}$-$C_{14}$)aminopropylmorpholine; et leurs mélanges.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant 8 à 22 atomes de carbone et contenant, au moins un groupe anionique hydrosolubilisant tel que, par exemple, un groupe carboxylate, sulfonate, sulfate, phosphate ou phosphonate, on peut citer encore les alkyl($C_8$-$C_{20}$)bétaïnes, les sulfobétaïnes, les alkyl($C_8$-$C_{20}$)amidoalkyl($C_6$-$C_8$)-bétaïnes ou les alkyl($C_8$-$C_{20}$)amidoalkyl($C_6$-$C_8$)sulfobétaïnes ; et leurs mélanges.

Parmi les dérivés d'amines, on peut citer les produits commercialisés sous la dénomination MIRANOL®, tels que décrits dans les brevets US-2,528,378 et US-2,781,354 et classés dans le dictionnaire CTFA, 3$^{ème}$ édition, 1982, sous les dénominations Amphocarboxy-glycinate et Amphocarboxypropionate de structures respectives (1) et (2) :

$$R_2\ \text{-}CONHCH_2CH_2\ \text{-}N^+(R_3)(R_4)(CH_2COO^-) \qquad (1)$$

dans laquelle :

- $R_2$ désigne représente un groupe alkyle dérivé d'un acide $R_2$-COOH présent dans l'huile de coprah hydrolysée, un groupe heptyle, nonyle ou undécyle,
- $R_3$ représente un groupe bêta-hydroxyéthyle, et
- $R_4$ représente un groupe carboxyméthyle ;
- Et

$$R_2\ \text{-}CONHCH_2CH_2\ \text{-}N(B)(C) \qquad (2)$$

dans laquelle :

- B représente $-CH_2CH_2OX'$,
- C représente $-(CH_2)_z$, -Y', avec z = 1 ou 2,
- X' représente le groupement $-CH_2CH_2$-COOH ou un atome d'hydrogène,
- Y' représente -COOH ou le groupe $-CH_2$ - CHOH - $SO_3H$,
- $R_2$ représente le groupe alkyle d'un acide $R_{2'}$-COOH présent dans l'huile de coprah ou dans l'huile de lin hydrolysée, un groupe alkyle, notamment en $C_{17}$ et sa forme iso, un groupe $C_{17}$ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium capryloamphodiacétate de disodium, cocoamphodipropionate de disodium, lauroamphodipropionate de disodium, caprylamphodipropionate

de disodium, capryloamphodipropionate de disodium, acide lauroamphodipropionique, acide cocoamphodipropionique.

A titre d'exemple on peut citer le cocoamphodiacétate commercialisé sous la dénomination commerciale MIRANOL® C2M concentré par la société RHODIA.

Parmi les tensioactifs amphothères, on utilise de préférence les alkyl($C_8$-$C_{20}$)bétaïnes telles que la cocobétaïne, les alkyl($C_8$-$C_{20}$)amidoalkyl($C_6$-$C_8$)bétaïnes telles que la cocamidobétaïne, les alkylamphodiacétates comme le cocoamphodiacétate disodique, et leurs mélanges.

La composition selon l'invention peut comprendre en outre un ou plusieurs tensioactifs cationiques bien connus en soi, tels que les sels d'amines grasses primaires, secondaires ou tertiaires, éventuellement polyoxyalkylénées, les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium, les dérivés d'imidazoline ; ou les oxydes d'amines à caractère cationique.

Les agents tensioactifs non ioniques, amphotères et cationiques décrits ci-dessus peuvent être utilisés seuls ou en mélanges et leur quantité est comprise entre 0,01% et 30% en poids, de préférence entre 0,05% et 20% en poids et mieux encore entre 0,1% et 10% en poids par rapport au poids total de la composition.

Les agents gélifiants et/ou épaississants différents des dérivés de cellulose et convenant pour les compositions de l'invention sont bien connus dans la technique et peuvent être choisis parmi les polymères et copolymères carboxyvinyliques, les polymères et copolymères (alkyl)acryliques, les polymères et copolymères (alkyl)acrylamides, les poly (oxyalkylène)glycols, les esters de poly(oxyalkylène)glycols, les alginates, les biosaccharides, les dérivés d'amidon, les gommes naturelles telles que les gommes de xanthane, de guar, de caroube, les scléroglucanes, les dérivés de chitine et de chitosane, les carraghénanes, les argiles, et leur mélanges.

A titre d'exemple d'agents gélifiants, notamment en phase aqueuse, on peut citer le SEPIGEL® 305 commercialisé par la société SEPPIC, le FUCOGEL® 1000 PP commercialisé par la société SOLABIA, le SYNTHALEN® K commercialisé par la société 3VSA, le LUVISKOL® VA 64 P commercialisé par la société BASF, l'HOSTACERIN® AMPS commercialisé par la société CLARIANT, le PEMULEN® TR1 commercialisé par la société GOODRICH, le LUBRAGEL® MS commercialisé par la société GUARDIAN, le SATIAGEL® KSO commercialisé par DEGUSSA et le KELTROL® commercialisé par la société KELCO, les SALCARE SC 95 ou SC 96 commercialisés par la société CIBA.

Les agents gélifiants additionnels représentent en général de 0,05 à 15%, de préférence de 0,5 à 10% en poids de la composition.

Les silicones utilisables en tant qu'additifs dans les compositions cosmétiques de la présente invention, sont des silicones volatiles ou non volatiles, cycliques, linéaires ou ramifiées, modifiées ou non par des groupements organiques, ayant une viscosité de $5.10^{-6}$ à $2{,}5 m^2/s$ à 25°C et de préférence $1.10^{-5}$ à $1 m^2/s$.

Les silicones utilisables conformément à l'invention peuvent être solubles ou insolubles dans la composition et en particulier être des polyorganosiloxanes insolubles dans la composition de l'invention. Elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:

(i) les silicones cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges. On peut également citer les cyclocopolymères du type diméthylsiloxanes/ méthylalkylsiloxane, tel que la SILICONE VOLATILE® FZ 3109 commercialisée par la société UNION CARBIDE, de formule :

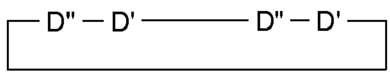

avec D'' : 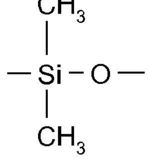 avec D' : 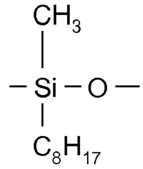

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange

d'octaméthylcyclotétrasiloxane et d'oxy-1,1'-(hexa-2,2,2',2',3,3'-triméthylsilyloxy) bis-néopentane ;

(ii) les silicones volatiles linéaires ayant 2 à 9 atomes de silicium et présentant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des silicones non volatiles, et plus particulièrement des polyalkylsiloxanes, des polyarylsiloxanes, des polyalkylarylsiloxanes, des gommes et des résines de silicones, des polyorganosiloxanes modifiés par les groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polyalkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 $mm^2/s$ ;
- les huiles VISCASIL® de GENERAL ELECTRIC et certaines huiles des séries SF (SF 96, SF 18) de GENERAL ELECTRIC.

On peut également citer les polyméthylsiloxanes à groupements terminaux diméthylsilanol connus sous le nom de dimethiconol (CTFA), tels que les huiles de la série 48 de la société RHODIA.

Dans cette classe de polyalkylsiloxanes, on peut également citer les produits commercialisés sous les dénominations "ABIL WAX® 9800 et 9801" par la société GOLDSCHMIDT qui sont des polyalkyl ($C_1$-$C_{20}$) siloxanes.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de $1.10^{-5}$ à $5.10^{-2} m^2/s$ à 25°C.

Parmi ces polyalkylarylsiloxanes on peut citer à titre d'exemple les produits commercialisés sous les dénominations suivantes :

- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polyorganosiloxanes, ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

On peut citer plus particulièrement les produits suivants :

- les gommes de polydiméthylsiloxane,
- les gommes de polydiméthylsiloxane/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane /diphénylsiloxane/ méthylvinylsiloxane.

Des produits plus particulièrement utilisables conformément à l'invention sont des mélanges tels que :

- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un

poids moléculaire moyen en nombre de 500 000 solubilisée dans l'huile SF 1202 Silicone Fluid correspondant au décaméthylcyclopentasiloxane ;

- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6} m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

$R_2SiO_{2/2}$, $R_3SiO_{1/2}$, $RSiO_{3/2}$ et $SiO_{4/2}$

dans lesquelles R représente un groupement hydrocarboné possédant 1 à 16 atomes de carbone ou un groupement phényle. Parmi ces produits, ceux particulièrement préférés sont ceux dans lesquels R désigne un groupe alkyle inférieur en $C_1$-$C_4$, plus particulièrement méthyle ou un groupe phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl ($C_{12}$)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939, ou DOW CORNING 2-8299 par la société DOW CORNING ou le produit commercialisé sous la dénomination BELSIL ADM LOG 1 par la société WACKER. Les groupements aminés substitués sont en particulier des groupements aminoalkyles en $C_1$-$C_4$ ;
- des groupements thiols, comme les produits commercialisés sous les dénominations GP 72A et GP 71 de GENESEE.
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.
- des groupements hydroxylés, comme les polyorganoxiloxanes à fonction hydroxyalkyle décrits dans la demande de brevet français FR-A- 8 516 334 ;
- des groupements alcoxyalkyle tels que, par exemple, les polyorganosiloxanes décrits dans le brevet US-A-4 957 732 ;
- des groupements anioniques du type carboxylique comme par exemple, dans le produits décrits dans le brevet EP 186 507 de la société CHISSO CORPORATION, ou de type alkyl-carboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations ABIL® S201 ET ABIL® S255 ;
- des groupements hydroxyacrylamino, comme les polyorganosiloxanes décrits dans la demande EP 342 834. On peut citer, par exemple, le produit Q2-8413 de la société DOW CORNING.

Les silicones telles que décrites ci-dessus peuvent être utilisées seules ou en mélange, en une quantité comprise entre 0,01 et 20% en poids, de préférence entre 0,1 et 5% en poids.

Les compositions de l'invention peuvent encore comporter des corps gras tels que les huiles minérales, végétales, animales et synthétiques, les cires, les esters gras, les alcools gras éthoxylés ou non éthoxylés, et les acides gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépin de raisin, de sésame, de noisette, d'abricot, de macadamia,

d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que Parléam® ; les isoparaffines comme l'isohexadécane et l'isodécane.
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M ;

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras saturés ou insaturés sont choisis plus particulièrement parmi l'acide myristique, l'acide palmitique, l'acide stéarique, l'acide béhénique, l'acide oléïque, l'acide linoléïque, l'acide linolénique et l'acide isostéarique.

Les esters gras sont notamment les esters d'acides carboxyliques en particulier les esters mono, di, tri ou tétracarboxyliques.

Les esters d'acides carboxyliques sont notamment les esters d'acides aliphatiques saturés ou insaturés, linéaires ou ramifiés en $C_1$-$C_{26}$ et d'alcools aliphatiques saturés ou insaturés, linéaires ou ramifiés en $C_1$-$C_{26}$, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en $C_4$-$C_{22}$ et d'alcools en $C_1$-$C_{22}$ et les esters d'acides mono di ou tricarboxyliques et d'alcools di, tri, tétra ou pentahydroxy en $C_2$-$C_{26}$.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle ; le dioctanoate de propylène glycol et le diheptanoate de néopentyl glycol. Les esters cités ci-dessus étant différents des esters de formule (I).

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

Comme alcools gras on peut citer les alcools gras saturés ou insaturés, linéaires ou ramifiés ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

Les corps gras représentent en général de 0,1 à 50%; de préférence 1 à 30%, et encore plus préférentiellement de 2 à 20% en poids de la composition totale.

Comme indiqué précédemment les compositions peuvent comprendre des polymères fixants

Tous les polymères fixants anioniques, cationiques, amphotères, non ioniques et leurs mélanges utilisés dans la technique peuvent être utilisés dans les compositions selon la présente demande.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule :

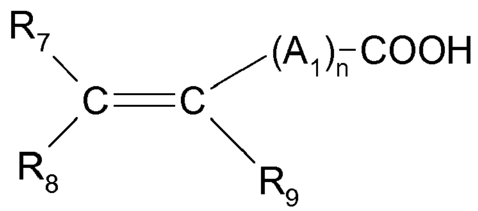

(I)

dans laquelle n est un nombre entier de 0 à 10, $A_1$ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, $R_7$ désigne un atome d'hydrogène, un groupement phényle ou benzyle, $R_8$ désigne un atome d'hydrogène, un groupement alkyle inférieur ou carboxyle, $R_9$ désigne un atome d'hydrogène, un groupement alkyle inférieur, un groupement $-CH_2-COOH$, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :

A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;

B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n$^{os}$ 75370 et 75371 ou proposés sous la dénomination QUADRAMER par la société AMERICAN CYANAMID. On peut également citer les copolymères d'acide acrylique et de méthacrylate d'alkyle en $C_1$-$C_4$ et les terpolymères de vinylpyrrolidone, d'acide acrylique et de méthacrylate d'alkyle en $C_1$-$C_{20}$, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF ;

On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL ;

C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique $\alpha$- ou $\beta$-cyclique. De tels polymères sont décrits entre autres dans les brevets français n$^{os}$ 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;

D) Les copolymères d'acides ou d'anhydrides carboxyliques monoinsaturés en $C_4$-$C_8$ choisis parmi :

- les copolymères comprenant (i) un ou plusieurs acides ou anhydrides maléique, fumarique, itaconique et (ii)

au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ® AN ou ES par la société ISP ;

- les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, $\alpha$-oléfine, esters acryliques ou méthacryliques, acides acrylique ou méthacrylique ou vinylpyrrolidone dans leur chaîne,

les fonctions anhydrides de ces copolymères étant éventuellement monoestérifiées ou monoamidifiées.
Ces polymères sont par exemple décrits dans les brevets français n^os^ 2 350 384 et 2 357 241 de la demanderesse ;
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :

- les sels de l'acide polyvinylsulfonique ayant une masse moléculaire comprise entre environ 1 000 et 100 000, ainsi que les copolymères avec un comonomère insaturé tel que les acides acrylique

ou méthacrylique et leurs esters, ainsi que l'acrylamide ou ses dérivés, les éthers vinyliques et la vinylpyrrolidone ;

- les sels de l'acide polystyrène-sulfonique tels que les sels de sodium vendus par exemple sous la dénomination Flexan® 500 et Flexan® 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.

Comme autre polymère fixant anionique utilisable selon l'invention, on peut citer le polymère anionique séquencé branché vendu sous la dénomination Fixate G-100 par la société NOVEON.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination Fixate G-100 par la société NOVEON.

Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination Fixate G-100 par la société NOVEON.

Les polymères filmogènes fixants cationiques utilisables selon la présente invention sont de préférence choisis parmi les polymères comportant des groupements amines primaires, secondaires, tertiaires et/ou quaternaires faisant

partie de la chaîne polymère ou directement reliés à celle-ci, et ayant un poids moléculaire compris entre 500 et environ 5 000 000 et de préférence entre 1 000 et 3 000 000.

Parmi ces polymères, on peut citer plus particulièrement les polymères cationiques suivants:

(1) les homopolymères ou copolymères dérivés d'esters ou d'amides acryliques ou méthacryliques et comportant au moins un des motifs de formules suivantes:

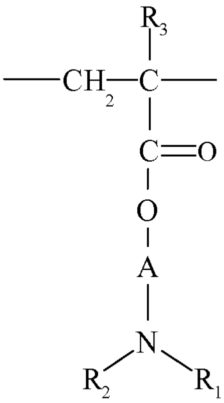
(A) ,
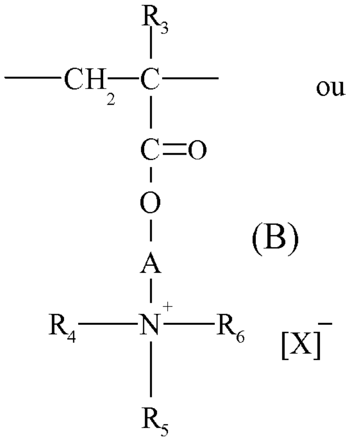
(B) ou
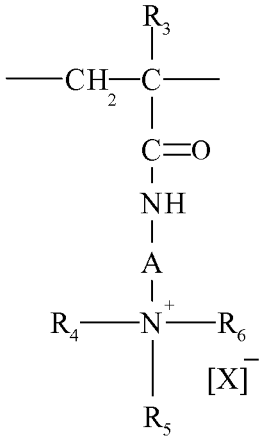
(C)

dans lesquelles:

R3 désigne un atome d'hydrogène ou un radical CH3;
A est un groupe alkyle linéaire ou ramifié comportant de 1 à 6 atomes de carbone ou un groupe hydroxyalkyle comportant de 1 à 4 atomes de carbone ;
R4, R5, R6, identiques ou différents, représentent un groupe alkyle ayant de 1 à 18 atomes de carbone ou un radical benzyle;
R1 et R2, identiques ou différents, représentent chacun un atome hydrogène ou un groupe alkyle ayant de 1 à 6 atomes de carbone;
X désigne un anion méthosulfate ou un halogénure tel que chlorure ou bromure.

Les copolymères de la famille (1) contiennent en outre un ou plusieurs motifs dérivant de comonomères pouvant être choisis dans la famille des acrylamides, méthacrylamides, diacétones-acrylamides, acrylamides et méthacrylamides substitués sur l'azote par des groupes alkyles inférieurs (C1-C4), des groupes dérivés des acides acryliques ou méthacryliques ou leurs esters, des vinyllactames tels que la vinylpyrrolidone ou le vinylcaprolactame, d'esters vinyliques.

Ainsi, parmi ces copolymères de la famille (1), on peut citer :

- les copolymères d'acrylamide et de méthacrylate de diméthylaminoéthyle quaternisé au sulfate de diméthyle ou avec un halogénure de diméthyle, tels que celui vendu sous la dénomination HERCOFLOC® par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium tel que celui vendu sous la dénomination RETEN par la société HERCULES,
- les copolymères vinylpyrrolidone/acrylate ou méthacrylate de dialkylaminoalkyle quaternisés ou non, tels que les produits vendus sous la dénomination "GAFQUAT® " par la société ISP comme par exemple "GAFQUAT® 734" ou "GAFQUAT® 755" ou bien les produits dénommés "COPOLYMER® 845, 958 et 937". Ces polymères sont décrits en détail dans les brevets français 2.077.143 et 2.393.573,
- les polymères à chaîne grasse et à motif vinylpyrrolidone, tels que les produits vendus sous la dénomination Stylèze W20 et Stylèze W10 par la société ISP,
- les terpolymères méthacrylate de diméthylaminoéthyle/vinylcaprolactame/vinylpyrrolidone tel que le produit commercialisé sous la dénomination GAFFIX VC 713 par la société ISP, et
- et les copolymères vinylpyrrolidone/méthacrylamide de diméthylaminopropyle quaternisés tels que les produits

commercialisés sous la dénomination "GAFQUAT® HS 100" par la société ISP.

(2) les polysaccharides cationiques, de préférence à ammonium quaternaire tels que ceux décrits dans les brevets américains 3.589.578 et 4.031.307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL.

(3) les copolymères quaternaires de vinylpyrrolidone et de vinylimidazole ;

(4) les chitosanes ou leurs sels; les sels utilisables sont en particulier les acétate, lactate, glutamate, gluconate ou le pyrrolidone-carboxylate de chitosane .

Parmi ces composés, on peut citer le chitosane ayant un taux de désacétylation de 90,5% en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL .

(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-$\alpha,\beta$-dicarboxylique dont l'un des groupements carboxyliques a été amené à réagir avec une polyamine comportant un ou plusieurs groupements amines primaires ou secondaires.

Les polymères fixants amphotères répondant à la définition donnée ci-dessus plus particulièrement préférés sont choisis parmi les polymères suivants :

(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain n° 3 836 537.

(2) les polymères comportant des motifs dérivant :

a) d'au moins un monomère choisi parmi les acrylamides ou les méthacrylamides substitués sur l'atome d'azote par un groupe alkyle,

b) d'au moins un comonomère acide contenant un ou plusieurs groupements carboxyliques réactifs, et

c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les composés dont les groupes alkyles comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléïque ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER® ou LOVOCRYL® 47 par la société NATIONAL STARCH.

(3) les polyaminoamides réticulés et acylés partiellement ou totalement dérivant de polyaminoamides de formule générale :

$$-\!\!\left[CO-R_{10}-CO-Z\right]\!\!- \qquad (II)$$

dans laquelle $R_{10}$ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :

a) dans les proportions de 60 à 100 % en moles, le groupe

$$-NH-\left[(CH_2)_x-NH\right]_p- \qquad (IV)$$

où x=2 et p=2 ou 3, ou bien x=3 et p=2

ce groupe dérivant de la diéthylène-triamine, de la triéthylène-tétraamine ou de la dipropylène-triamine;

b) dans les proportions de 0 à 40 % en moles, le groupe (IV) ci-dessus, dans lequel x=2 et p=1 et qui dérive de l'éthylène-diamine, ou le groupe dérivant de la pipérazine :

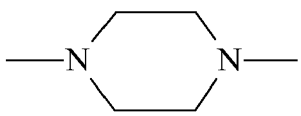

c) dans les proportions de 0 à 20 % en moles, le groupe $-NH-(CH_2)_6-NH-$ dérivant de l'hexaméthylènediamine,

ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.

Les alcane-sultones utilisées dans l'acylation sont de préférence la propane- ou la butane-sultone, les sels des agents d'acylation sont de préférence les sels de sodium ou de potassium.

(4) les polymères comportant des motifs zwittérioniques de formule :

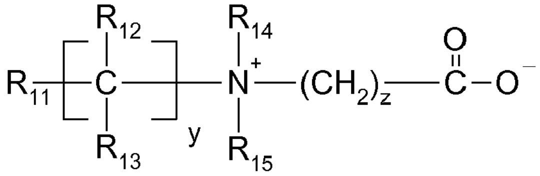

(IV)

dans laquelle $R_{11}$ désigne un groupement insaturé polymérisable tel qu'un groupement acrylate, méthacrylate, acrylamide ou méthacrylamide, y et z représentent un nombre entier de 1 à 3, $R_{12}$ et $R_{13}$ représentent un atome d'hydrogène, un groupe méthyle, éthyle ou propyle, $R_{14}$ et $R_{15}$ représentent un atome d'hydrogène ou un groupe alkyle de telle façon que la somme des atomes de carbone dans $R_{14}$ et $R_{15}$ ne dépasse pas 10.

Les polymères comprenant de tels motifs peuvent également comporter des motifs dérivés de monomères non

zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.

A titre d'exemple, on peut citer les copolymères méthacrylate de méthyle/diméthyl-carboxyméthylammonio-éthylméthacrylate de méthyle, tel que le produit vendu sous la dénomination DIAFORMER Z301 par la société SANDOZ.

(5) les polymères dérivés du chitosane comportant des motifs monomères répondant aux formules suivantes :

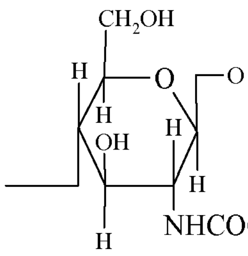
(D)

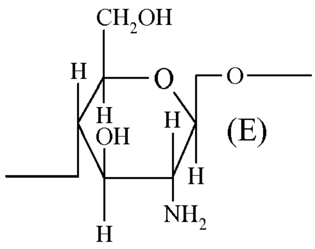
(E)

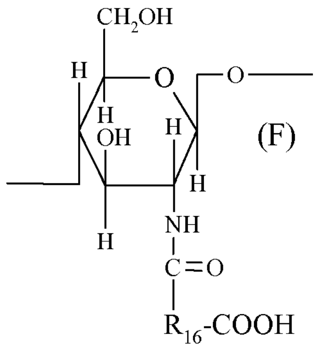
(F)

le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), $R_{16}$ représente un groupe de formule :

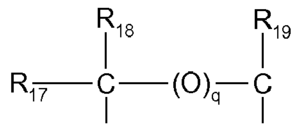

dans laquelle si q=0, $R_{17}$, $R_{18}$ et $R_{19}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes $R_{17}$, $R_{18}$ et $R_{19}$ étant dans ce cas un atome d'hydrogène ;

ou si q=1, $R_{17}$, $R_{18}$ et $R_{19}$ représentent chacun un atome d'hydrogène, ainsi que les sels formés par ces composés avec des bases ou des acides.

(6) Les polymères répondant à la formule générale (V) sont, par exemple, décrits dans le brevet français 1 400 366 :

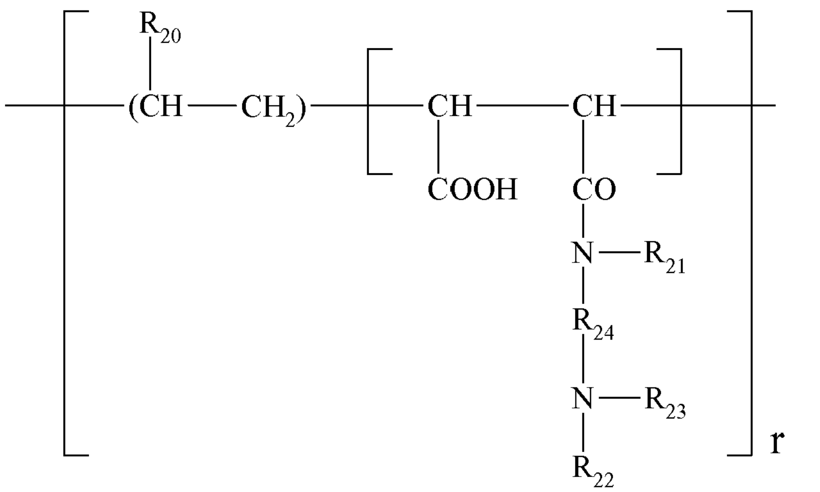
(V)

dans laquelle $R_{20}$ représente un atome d'hydrogène, un groupe $CH_3O$, $CH_3CH_2O$, phényle, $R_{21}$ désigne un atome

d'hydrogène ou un groupe alkyle inférieur tel que méthyle, éthyle, $R_{22}$ désigne un atome d'hydrogène ou un groupe alkyle inférieur en $C_1$-$C_6$ tel que méthyle, éthyle, $R_{23}$ désigne un groupe alkyle inférieur en $C_1$-$C_6$ tel que méthyle, éthyle ou un groupe répondant à la formule : $-R_{24}-N(R_{22})_2$, $R_{24}$ représentant un groupement $-CH_2-CH_2-$, $-CH_2-CH_2-CH_2-$, $-CH_2-CH(CH_3)-$, $R_{22}$ ayant les significations mentionnées ci-dessus.

(7) Les polymères dérivés de la N-carboxyalkylation du chitosane comme le N-carboxyméthyl-chitosane ou le N-carboxybutyl-chitosane, vendus sous la dénomination « EVALSAN » par la société JAN DEKKER.

(8) Les polymères amphotères du type -D-X-D-X choisis parmi:

a) les polymères obtenus par action de l'acide chloracétique ou le chloracétate de sodium sur les composés comportant au moins un motif de formule :

-D-X-D-X-D- (VI)

où D désigne un groupe

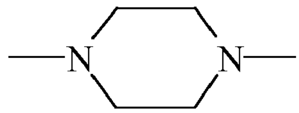

et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.

b) Les polymères de formule :

-D-X-D-X- (VI')

où D désigne un groupe

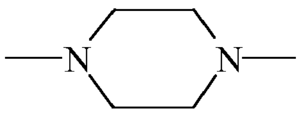

et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyles et comportant un ou plusieurs atomes d'azote, l'atome d'azote étant substitué par une chaîne alkyle interrompue éventuellement par un atome d'oxygène et comportant obligatoirement une ou plusieurs fonctions carboxyles ou une ou plusieurs fonctions hydroxyles et bétaïnisées par réaction avec l'acide chloracétique ou du chloracétate de soude.

(9) les copolymères alkyl($C_1$-$C_5$)vinyléther/anhydride maléique modifiés partiellement par semiamidification avec une N,N-dialkylaminoalkylamine telle que la N,N-diméthylaminopropylamine ou par semiestérification avec un N, N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthylméthacrylate de méthyle vendu par exemple sous la dénomination DIAFORMER Z301 par la société SANDOZ.

Les polymères fixants non ioniques utilisables selon la présente invention sont choisis, par exemple, parmi :

- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth) acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyles des polymères non ioniques mentionnés ci-dessus ont, de préférence, de 1 à 6 atomes de carbone.
Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.
Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.
Ces polymères peuvent être amphotères, anioniques ou non ioniques, et ils sont de préférence anioniques ou non ioniques.
De tels polymères sont, par exemple, les copolymères susceptibles d'être obtenus par polymérisation radicalaire à partir du mélange de monomères formé :

a) de 50 à 90 % en poids d'acrylate de tertiobutyle,
b) de 0 à 40 % en poids d'acide acrylique,
c) de 5 à 40 % en poids d'un macromère siliconé de formule

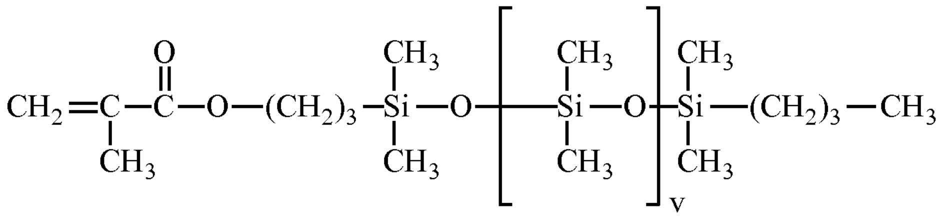

où v est un nombre allant de 5 à 700, les pourcentages en poids étant calculés par rapport au poids total des monomères.
D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS)

sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex® Silk commercialisé par la société BASF.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR® et LUVISET® Si PUR par la société BASF.

La concentration en polymère(s) fixant(s) utilisée dans les compositions selon la présente invention est comprise entre 0,1 et 20%, de préférence entre 0,5 et 10% en poids par rapport au poids total de la composition.

L'homme du métier saurait ajouter les additifs sans perturber les propriétés des compositions de l'invention.

Les compositions selon l'invention peuvent se présenter sous toutes les formes appropriées pour une application topique, notamment sous forme de solutions du type lotion ou sérum ; sous forme de gels aqueux ; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses ; sous forme de mousse, sous forme de spray, ou d'aérosol ; ou encore sous forme de brosse ou de stick.

De préférence, les compositions se présentent sous forme de gels. De préférence les compositions ont une viscosité supérieure à 500 cps à la température de 25°C et à un taux de cisaillement de $1s^{-1}$.

Lorsque la composition selon l'invention est conditionnée dans un dispositif aérosol, elle comprend au moins un agent propulseur, qui peut être choisi parmi les hydrocarbures volatiles, tels que le N-butane, le propane, l'isobutane, le pentane, les hydrocarbures halogénés et leurs mélanges. On peut également utiliser en tant que propulseur le gaz carbonique, le protoxyde d'azote, le diméthyléther (DME), l'azote, ou l'air comprimé. On peut aussi utiliser des mélanges de propulseurs. De préférence, on utilise le diméthyl éther.

Avantageusement, l'agent propulseur est présent à une concentration comprise entre 5 et 90% en poids par rapport au poids total de la composition dans le dispositif aérosol, et, plus particulièrement, à une concentration comprise entre 10 et 60%.

La composition selon l'invention peut notamment être utilisée en application non rincée sur les cheveux.

L'invention a également pour objet un procédé de traitement des matières kératiniques comprenant l'application d'une composition cosmétique selon l'invention, et un procédé de mise en forme des cheveux, comprenant l'application d'une composition cosmétique selon l'invention. En particulier, l'invention concerne un procédé de coiffage comprenant l'application d'une composition selon l'invention sur les cheveux, le rinçage éventuel des cheveux, puis la mise en forme et le séchage des cheveux.

Les exemples qui suivent illustrent l'invention sans en limiter la portée.

## Exemples

Les compositions suivantes ont été réalisées :

Les concentrations sont exprimées en gramme de matières actives pour 100 grammes de composition.

| | Composition A | Composition B | Composition C | Composition D |
|---|---|---|---|---|
| Polyuréthane Cationique à séquence polyoléfine (1) | 4,2 | 5,8 | 2 | 1,5 |
| Hydroxypropyl Méthylcellulose (5) | 1 | 3 | - | - |
| Cetyl Hydroxyethyl Cellulose (2) | - | - | 2 | 3 |
| mélange de polyquaternium-37 (3) | 1,50 | - | - | - |
| PEG/PPG-17/18 Dimethicone (4) | 1 | - | - | - |
| Propylene glycol | 2,5 | 5 | 2,5 | 2 |
| glycérol | - | - | - | 2 |
| Huile de ricin hydrogénée - PEG 40 (6) | | 5 | 5,5 | 4 |
| Isoceteth-20 (9) | 3 | - | - | 1,8 |

(suite)

| | Composition A | Composition B | Composition C | Composition D |
|---|---|---|---|---|
| parfum | 0,3 | 0,5 | - | 0,3 |
| alcool | - | - | - | 5 |
| conservateur | 0,9 | 0,7 | 0,7 | 0,7 |
| Eau | Qsp 100 | Qsp 100 | Qsp 100 | Qsp 100 |

(1) polyuréthane issu de la polycondensation de KRASOL LBHP® 2000 (67,9%), de N-méthyldiéthanolamine (8,7%) et de isophoronediisocyanate (23,4%).
(2) POLYSURF 67 CS (Aqualon)
(3) SALCARE SC 96 (CIBA)
(4) Q2-5220 (DOW CORNING)
(5) METHOCEL F 4M (DOW CHEMICAL)
(6) CREMOPHOR CO 40 (BASF)
(7) Ucare Polymer JR30M (AMERCHOL)
(8) PEG 6000 Distearate (AKZO NOBEL)

Les compositions ci-dessus présentent une consistance adéquate permettant une application facile et une localisation aisée sur des cheveux propres. Après séchage la chevelure présente un maintien naturel de longue durée.

Exemple comparatif

| | Composition I (invention) | Composition II (comparative) |
|---|---|---|
| Polyuréthane cationique à sequence polyoléfine(1) | 6.78 | 6.78 |
| Polyquaternium-10(7) | 1.5 | |
| Huile de ricin hydrogénée PEG 40(6) | 5 | 5 |
| PEG 150 Distearate(8) | 1.5 | 1.5 |
| PEG/PPG-17/18 | | |
| Dimethicone (4) | | |
| Propylène glycol | 2.5 | 2.5 |
| Conservateurs | Qs | Qs |
| Eau | Qsp 100 | Qsp 100 |

On a comparé la composition I à la composition II. La composition I selon l'invention s'applique plus aisément sur les cheveux. Elle s'étale plus facilement et se localise plus facilement.

On obtient avec la composition de l'invention un maintien tout à fait naturel.

## Revendications

1. Composition cosmétique comprenant, dans un milieu cosmétiquement acceptable :

   (i) un ou plusieurs polyuréthanes cationiques comportant un ou plusieurs motifs non ioniques dérivés d'un homo- et/ou copolymère d'oléfine, et
   (ii) une ou plusieurs celluloses cationiques ou non ioniques.

2. Composition cosmétique selon la revendication 1, **caractérisée en ce que** 50% en poids ou plus des motifs non ioniques du polyuréthane dérivent d'un homopolymère ou copolymère d'oléfine.

**3.** Composition cosmétique selon l'une quelconque des revendications 1 ou 2, **caractérisée en ce** les homopolymères et copolymères d'oléfines sont des homopolymères et copolymères portant à leurs extrémités des fonctions à hydrogène labile, et présentant des motifs choisis parmi les motifs d'éthylène, de propylène, de 1-butylène, de 2-butylène, d'isobutylène, de 1,2-butadiène, de 1,4-butadiène, d'isoprène et leurs mélanges.

**4.** Composition cosmétique selon l'une quelconque des revendications précédentes **caractérisée en ce que** le polyuréthane cationique comprend:

(a) des motifs cationiques résultant de la réaction d'une ou plusieurs amines tertiaires ou quaternaires présentant au moins deux fonctions réactives à hydrogène labile,
(b) des motifs non ioniques dont au moins un motif (b1) résulte de la réaction d'au moins un polymère choisi parmi les homopolymères et les copolymères d'oléfines portant à leurs extrémités des fonctions réactives à hydrogène labile et ayant une température de transition vitreuse (Tg), mesurée par analyse enthalpique différentielle, inférieure à 10°C, et
(c) des motifs résultant de la réaction d'un ou plusieurs diisocyanates.

**5.** Composition cosmétique selon la revendication 4, **caractérisée en ce que** les motifs cationiques (a) résultent de la réaction de la N-méthyldiéthanolamine ou de la N-tert-butyldiéthano lamine.

**6.** Composition cosmétique selon l'une quelconque des revendications 4 ou 5, **caractérisée en ce que** le polyuréthane cationique contient éventuellement au moins un motif non ionique (b2), différent du motif (b1), dérivant d'un composé non ionique monomère contenant au moins deux fonctions à hydrogène labile capables de réagir avec le ou lesdits composés (c) contenant au moins un diisocyanate.

**7.** Composition cosmétique selon la revendication 6, **caractérisée en ce que** les motifs cationiques (a) représentent de 0,1 à 90%, de préférence de 1 à 30%, mieux de 1 à 20% en poids, les motifs non-ioniques dérivés d'un homo- ou copolymère d'oléfine (b1) représentent de 10 à 99,9%, de préférence de 20 à 99% et mieux de 30 à 85% en poids, et les motifs non-ioniques (b2) représentent de 0 à 50%, de préférence 0 à 30% en poids du total des motifs du polyuréthane cationique.

**8.** Composition cosmétique selon l'une quelconque des revendications précédentes **caractérisée en ce que** le diisocyanate est choisi parmi le méthylènediphényldiisocyanate, le méthylènecyclohexane-diisocyanate, l'isophoronediisocyanate, le toluènediisocyanate, le naphtalènediisocyanate, le 1,4-butanediisocyanate et le 1,6-hexanediisocyanate.

**9.** Composition cosmétique selon l'une quelconque des revendications précédentes, **caractérisée en ce que** les motifs (c) représentent de 1 à 60%, de préférence de 5 à 50%, et idéalement 10 à 40% du poids total des motifs du polyuréthane cationique.

**10.** Composition cosmétique selon l'une quelconque des revendications précédentes, **caractérisée en ce que** le polyuréthane cationique ne comprend pas de motifs en plus des motifs (a), (b) et (c).

**11.** Composition cosmétique selon l'une quelconque des revendications précédentes, **caractérisée en ce que** le (ou les) polyuréthane(s) cationique(s) représente(nt) de 0,01% à 40%, de préférence 0,05 à 20% et idéalement de 0,1 à 10% en poids rapporté à la composition cosmétique finale.

**12.** Composition cosmétique selon l'une quelconque des revendications précédentes **caractérisée en ce que** les celluloses sont des hydroxyalkylcelluloses et de préférence des hydroxyéthylcelluloses ou des hydroxypropylcelluloses.

**13.** Composition cosmétique selon l'une quelconque des revendications précédentes **caractérisée en ce que** les celluloses sont choisies parmi les celluloses ne comportant pas de chaîne hydrophobe et les celluloses comportant une ou plusieurs chaînes hydrophobes.

**14.** Composition cosmétique selon l'une quelconque des revendications précédentes, **caractérisée en ce que** la quantité en celluloses cationiques ou non-ioniques va de 0,01 à 50 % en poids, de préférence de 0,1 à 20 %, encore plus préférentiellement de 0,5 à 10% en poids, rapporté au poids de la composition cosmétique finale.

**15.** Procédé de coiffage comprenant l'application d'une composition selon l'une quelconque des revendications 1 à 14 sur les cheveux, le rinçage éventuel des cheveux, puis la mise en forme et le séchage des cheveux.

Europäisches Patentamt
European Patent Office
Office européen des brevets

# RAPPORT DE RECHERCHE EUROPEENNE

Numéro de la demande
EP 09 15 8013

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 1 837 353 A (OREAL [FR]) 26 septembre 2007 (2007-09-26) * le document en entier * ----- | 1,15 | INV. A61K8/73 A61K8/87 A61Q5/04 |
| A | US 6 335 003 B1 (KIM SON NGUYEN [DE] ET AL) 1 janvier 2002 (2002-01-01) * le document en entier * ----- | 1-15 | |

DOMAINES TECHNIQUES RECHERCHES (IPC)
A61K

Le présent rapport a été établi pour toutes les revendications

1

| Lieu de la recherche | Date d'achèvement de la recherche | Examinateur |
|---|---|---|
| Munich | 10 juin 2009 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 09 15 8013

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-06-2009

| Document brevet cité au rapport de recherche | Date de publication | Membre(s) de la famille de brevet(s) | Date de publication |
|---|---|---|---|
| EP 1837353 A | 26-09-2007 | BR PI0700861 A | 27-11-2007 |
| | | CN 101270182 A | 24-09-2008 |
| | | FR 2898603 A1 | 21-09-2007 |
| | | JP 2007254740 A | 04-10-2007 |
| | | KR 20070095248 A | 28-09-2007 |
| | | US 2007283977 A1 | 13-12-2007 |
| US 6335003 B1 | 01-01-2002 | CA 2148805 A1 | 23-06-1994 |
| | | DE 4241118 A1 | 09-06-1994 |
| | | WO 9413724 A1 | 23-06-1994 |
| | | EP 0672076 A1 | 20-09-1995 |
| | | ES 2108415 T3 | 16-12-1997 |
| | | JP 8504454 T | 14-05-1996 |
| | | JP 3369180 B2 | 20-01-2003 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9413724 A
- EP 0619111 A
- FR 2815350
- FR 2833960
- FR 1492597
- US 4131576 A
- US 2528378 A
- US 2781354 A
- FR 8516334 A
- US 4957732 A
- EP 186507 A
- EP 342834 A
- JP 2295912 A
- FR 1222944
- DE 2330956
- FR 1580545
- FR 2265782
- FR 2265781
- FR 1564110
- FR 2439798
- US 2047398 A
- US 2723248 A
- US 2102113 A
- GB 839805 A
- FR 2350384
- FR 2357241
- FR 2198719
- US 4128631 A
- EP 080976 A
- FR 2077143
- FR 2393573
- FR 1400366
- EP 0412704 A
- EP 0412707 A
- EP 0640105 A
- WO 9500578 A
- EP 0582152 A
- WO 9323009 A
- US 4693935 A
- US 4728571 A
- US 4972037 A
- EP 0751162 A
- EP 0637600 A
- EP 0648485 A
- FR 2743297
- EP 0656021 A
- WO 9403510 A


### Littérature non-brevet citée dans la description


- **Walter NOLL.** Chemistry and Technology of Silicones. Academie Press, 1968
- **TODD ; BYERS.** Volatile Silicone fluids for cosmetics. *Cosmetics and Toiletries,* Janvier 1976, vol. 91, 27-32